# EUROPÄISCHE PATENTANMELDUNG

(11) **EP 1 825 888 A1**
(43) Veröffentlichungstag der Anmeldung: **29.08.2007**
(21) Anmeldenummer: 06110466.7
(22) Anmeldetag: 28.02.2006
(51) Int. Cl.: A63B 24/00

(54) **Vorrichtung zur Einstellung der Belastungsstärke eines Ergometers, Ergometer und Verfahren zur Regelung eines Ergometers**

(71) Anmelder: Schiller AG, 6340 Baar (CH)
(72) Erfinder: Schiller, Alfred, 8914, Aeugst a.A. (CH)
(74) Vertreter: Hepp, Dieter

(57) **Zusammenfassung**

Die Erfindung betrifft eine Vorrichtung (2) zur Einstellung der Belastungsstärke eines Ergometers (1), ein Ergometer (1) und ein Verfahren zur Regelung eines Ergometers, wobei die Belastungsstärke des Ergometers (1) aufgrund von Gaskonzentrationswerten in der Atemluft regelbar ist. Gaskonzentrationswerte sind Parameter, die ein umfassendes und direktes Mass für die physiologische Befindlichkeit des Trainierenden, insbesondere für die Effizienz des Stoffwechsels, sind.

## Beschreibung

Die Erfindung betrifft eine Vorrichtung zur Einstellung der Belastungsstärke eines Ergometers, ein Ergometer und ein Verfahren zur Regelung eines Ergometers.

Bei einem Ergometer handelt es sich um ein Gerät, mit dem körperliche Kraft oder Arbeit gemessen werden kann und mit dem ein Trainingsprogramm unter definierten und kontrollierten Bedingungen durchgeführt werden kann. Ergometer gibt es in unterschiedlichsten Varianten, z.B. in Fahrradform, wobei in der Regel eine Schwungscheibe durch Pedalantrieb in Bewegung gebracht wird. Gebräuchlich sind ausserdem Laufbänder und Rudergeräte.

Die Belastungsstärke des Ergometers, zum Beispiel der Bremswiderstand beim Fahrradergometer, ist einstellbar oder wird in Abhängigkeit von Messdaten geregelt.

Aus dem Stand der Technik ist beispielsweise eine Pulsregelung bekannt, wobei der Trainingspuls des Benutzers durch automatische Lastregelung konstant gehalten wird. Der Puls sagt aber nichts aus über die Effizienz des Stoffwechsels und ist daher nur ein sekundärer Anhaltspunkt für die Leistungsfähigkeit.

Aus DE 202 19 433 U1 ist ein Ergometer bekannt, das eine steuerbare Bremsvorrichtung aufweist und die Steuereinheit mit einer Überwachungseinrichtung für die Sauerstoffsättigung verbunden ist. Die Sauerstoffsättigung wird mittels Pulsoximetrie über eine Lichtmesssonde an einem Kapillarblut führenden Körperteil gemessen.

Die Pulsoximetrie ist eine Messmethode, die Auskunft über den Sauerstoffgehalt im arteriellen Blut gibt. Es handelt sich um eine indirekte Messmethode, die einen Farbunterschied bei unterschiedlicher Sauerstoffsättigung ausnutzt. Die Messmethode ist zwar einfach, besitzt aber prinzipielle Grenzen. Die Messgenauigkeit kann durch Bewegungsartefakte, Minderperfusion des Messortes, Dyshämoglobine (vor allem bei Rauchern eine Fehlerquelle) und andere Faktoren eingeschränkt werden. Für eine möglichst präzise Messung muss das Messgerät überdies empirisch kalibriert werden.

Die Sauerstoffsättigung im arteriellen Blut eines Körperteils repräsentiert zudem nur ein Teilaspekt des Stoffwechsels.

Die Aufgabe der Erfindung besteht somit darin, die Nachteile des Bekannten zu überwinden und insbesondere eine Vorrichtung zur Einstellung der Belastungsstärke eines Ergometers, ein Ergometer und ein Verfahren zur Einstellung der Belastungsstärke eines Ergometers zu schaffen, welche eine möglichst präzise Berücksichtigung der physiologischen Vorgänge ermöglicht.

Die Aufgabe wird gelöst durch eine Vorrichtung zur Einstellung der Belastungsstärke eines Ergometers, mit welcher die Belastungsstärke des Ergometers aufgrund von Gaskonzentrationswerten in der Atemluft regelbar ist. Die Gaskonzentrationswerte in der Atemluft sind Parameter, welche ein umfassendes und direktes Mass für die physiologische Befindlichkeit des Trainierenden sind.

Bei einem optimierten Training kommt es weniger darauf an, dass der Trainierende eine bestimmte am Trainingsgerät vorgewählte Last überwindet, als vielmehr darauf, dass er sein eigenes Leistungsspektrum ausschöpft. Die Belastungsstärke wird deshalb aufgrund seiner physiologischen Befindlichkeit geregelt, die von individuellen Faktoren aber auch von äusseren Einflüssen, wie z.B. der Umgebungstemperatur, der Höhe über Meeresspiegel und anderem, abhängt.

Die erfindungsgemässe Vorrichtung entspricht einer Regeleinrichtung mit einem Regelkreis, dem als Sollwert oder Führungsgrösse eine Gaskonzentration oder ein davon abgeleiteter Wert vorliegt, und der als Stellgrösse ein Signal erzeugt, das die Belastungsstärke des Ergometers einstellt.

Gaskonzentrationswerte in Atemluft sind zumeist einfach erfassbar, es ist kein medizinisch geschultes Personal notwendig, wie es beispielsweise bei der Bestimmung von Gaskonzentrationen in Blut der Fall sein könnte.

Vorteilhafterweise umfasst die erfindungsgemässe Vorrichtung daher eine Messeinrichtung, welche die Gaskonzentrationswerte, typischerweise O₂ und CO₂, in der Atemluft erfasst.

Gaskonzentrationswerte der Atemluft werden nicht invasiv gemessen, sie können kontinuierlich oder diskontinuierlich mit offenen oder geschlossenen Respiratorsystemen bestimmt werden.

Die Vorrichtung zur Einstellung der Belastungsstärke und die Messeinrichtung kann in einer baulichen Einheit zusammengefasst werden, sodass ein kompaktes Gerät entsteht, das sich für Ergometer in vielfältigen Bereichen einsetzen lässt.

In einer Ausführung der Erfindung ist die Messeinrichtung mit einer Atemmaske ausgestattet. Diese kann von dem Trainierenden angelegt werden und begrenzt das Volumen des zu analysierenden Gases. Die Messung kann schneller erfolgen und ist durch die Abgrenzung zum Aussenraum weniger störanfällig.

Es können während des Ergometerbetriebs periodisch Messungen durchgeführt werden, anhand deren Resultate die Belastungsstärke des Ergometers geregelt wird. Die Gaskonzentrationswerte können auch kontinuierlich gemessen und an die Vorrichtung übermittelt werden. In diesem Fall findet eine so genannte "Breath-by-Breath" Gasanalyse statt und die Ergebnisse jedes einzelnen Atemzuges haben Einfluss auf die Regelung des Ergometers. Durch die kontinuierliche Kontrolle und Übermittlung der Gaskonzentrationen kann der physiologische Zustand der Trainingsperson optimal an eine Vorgabe angepasst werden.

Der Stoffwechsel kann durch eine Messung von Sauerstoffverbrauch und Kohlendioxidproduktion erfasst werden.

Vorteilhafterweise sind an der erfindungsgemässen Vorrichtung daher mindestens Messmittel zur Bestimmung des Sauerstoffgehalts und/oder des Kohlendioxidgehalts der eingeatmeten und ausgeatmeten Luft vorgesehen.

Der Sauerstoffgehalt kann mit einer chemischen O₂ Zelle gemessen werden, der Kohlendioxidgehalt mit einem Ultraschall CO₂ Analysator. Die Anstiegszeiten solcher Messsensoren müssen sehr Schnell sein, insbesondere kleiner als 150 ns, um bei jedem Atemzug Auswertungen vorzunehmen.

Dabei sind nicht die absoluten Gaskonzentrationen oder die absoluten Differenzen der Gaskonzentrationen das gewünschte Mass. Die Leistungsfähigkeit des Trainierenden lässt sich an den Differenzen der Gaskonzentrationen, also dem Gasumsatz, pro einem bestimmten Zeitintervall ablesen. In einer vorteilhaften Ausführung der erfindungsgemässen Vorrichtung ist daher der Gasumsatz pro Zeitintervall bestimmbar.

Für ein Ausdauertraining kann der Sollwert oder die Führungsgrösse zum Beispiel die Maximale Sauerstoffaufnahme des Trainierenden sein, die angibt, wieviel Milliliter Sauerstoff der Körper im Zustand der Ausbelastung maximal pro Minute verwerten kann. Die erfindungsgemässe Vorrichtung bestimmt dann den Sauerstoffumsatz und regelt die Belastungsstärke so, dass die maximale Sauerstoffaufnahme erreicht wird.

Oft wird die auf das Körpergewicht bezogene relative maximale Sauerstoffaufnahme verwendet (Angabe in ml O₂/min/kg Körpergewicht), da ihre Aussagekraft bezüglich der Bewertung der Ausdauerleistungsfähigkeit für Sportarten, bei denen das Eigengewicht des Körpers eine Rolle spielt, wie Laufen oder Radfahren, höher ist.

Es kann als Sollgrösse für die Regelung auch ein bestimmter Kalorienverbrauch einstellbar sein. Aus der Sauerstoffaufnahme (VO₂) lässt sich eine metabolische Einheit "MET" errechnen. 1 MET entspricht der O₂-Aufnahme einer erwachsenen Person im Sitzen, in der Regel 3.5 ml VO₂ pro Minute und kg Körpergewicht. Das Energieäquivalent von 1 MET ist ungefähr 1 kcal pro kg Körpergewicht und Stunde.

Ein vorgewählter Energieverbrauch kann also über die Belastungsstärke bei einer entsprechenden Saustoffaufnahme geregelt werden.

Die Vorrichtung kann über fest eingestellte oder auswählbare Sollwerte verfügen. In einer bevorzugten Ausführung der Erfindung weist die Vorrichtung eine Eingabemöglichkeit für Sollwerte und/oder Sollwertkurven und/oder Grenzwerte auf. Die Sollwerte können individuell für den Trainierenden vorbestimmt werden. Es kann auch eine definierter Trainingsverlauf festgelegt werden, der zum Beispiel eine Leistungssteigerung anregt oder Trainingspausen enthält. Dazu wird in die Einrichtung eine entsprechende Sollwertkurve eingegeben.

Zusätzlich können Grenzwerte eingegeben werde, die nach Möglichkeit nicht unter- oder überschritten werden sollen. Die Vorrichtung kann über einen Warnsignalgeber verfügen, durch den ein akustisches oder optisches Warnsignal bei Über- oder Unterschreitung von Grenzwerten erzeugbar ist.

Vorteilhafterweise verfügt die Vorrichtung über mindestens eine Anzeige für die Gaskonzentrationswerte und/oder davon abgeleitete Grössen. Der Trainierende oder ein Betreuer kann die erbrachte Leistung dann selbst überwachen.

Die Vorrichtung kann zusätzlich über Mittel zum Abspeichern der gemessenen Werte und des Trainingsverlaufs verfügen und/oder über eine Schnittstelle zum Datentransfer.

Vorteilhafterweise kann die Vorrichtung noch weitere Parameter erfassen und verarbeiten, beispielsweise die Herzfrequenz, den Blutdruck, EKG Daten, das Atemvolumen, die Atemfrequenz und/oder die Atemstromstärke. Die weiteren Parameter können lediglich mit erfasst werden. Sie können auch zusätzlich oder alternativ zur Regelung der Belastungsstärke verwendet werden.

Die Aufgabe wird ausserdem durch ein Ergometer mit einer Vorrichtung zum Einstellen der Belastungsstärke, insbesondere gemäss den oben beschriebenen Eigenschaften, gelöst, wobei die Belastungsstärke des Ergometers aufgrund von Gaskonzentrationswerten in der Atemluft regelbar ist.

Die Vorrichtung zur Regelung der Belastungsstärke kann dabei in das Ergometer integriert sein.

In einer vorteilhaften Ausführung weist die Vorrichtung um Einstellen der Belastungsstärke eine Schnittstelle zu einer Messeinrichtung für Gaskonzentrationswerte in der Atemluft auf. Die Vorrichtung kann somit auf Bedarf oder kontinuierlich gemessene Gaskonzentrationswerte oder davon abgeleitete Werte empfangen und diese zur Regelung verwenden.

Besonders bevorzugt ist ein Ergometer mit einer Vorrichtung zum Einstellen der Belastungsstärke und einer Messeinrichtung für Gaskonzentrationswerte in der Atemluft ausgestattet. Die Vorrichtung zum Einstellen der Belastungsstärke und die Messeinrichtung können als bauliche Einheit in das Ergometer integriert sein. Eine komplizierte Verkabelung zwischen den Geräten ist nicht notwendig, was die Bedienung und den Transport erleichtert.

Das Ergometer kann alternativ oder zusätzlich mit Vorrichtungen zum Einstellen der Belastungsstärke ausgestattet werden, die eine Regelung aufgrund anderer physiologischer Parameter, wie Blutdruck oder Puls, vornehmen.

Die Aufgabe wird ausserdem gelöst durch ein Verfahren zur Regelung eines Ergometers mit den Verfahrensschritten (i) Messen von Gaskonzentrationswerten in der Atemluft und (ii) Regeln der Belastungsstärke aufgrund der Gaskonzentrationswerte. Die Messung erfolgt vorteilhafterweise bei jedem Atemzug, sodass eine kontinuierliche Regelung möglich ist.

In einer vorteilhaften Ausführung des Verfahrens wird zusätzlich der Gasumsatz pro Zeitintervall bestimmt und diese von den Gaskonzentrationswerten abgeleitete Grösse als Regelgrösse verwendet.

Sollwerte können in einem Belastungstest ermittelt werden, wobei Belastungstests und die Messung der Werte an demselben Ergometer ausgeführt werden können.

Die Erfindung ist in Folgenden in Ausführungsbeispielen anhand von Zeichnungen näher erläutert.

Es zeigt
- Figur 1: ein Beispiel für ein Ergometer mit einer Vorrichtung zum Einstellen der Belastungsstärke;
- Figur 2: eine schematische Darstellung eines erfindungsgemässen Regelkreises.

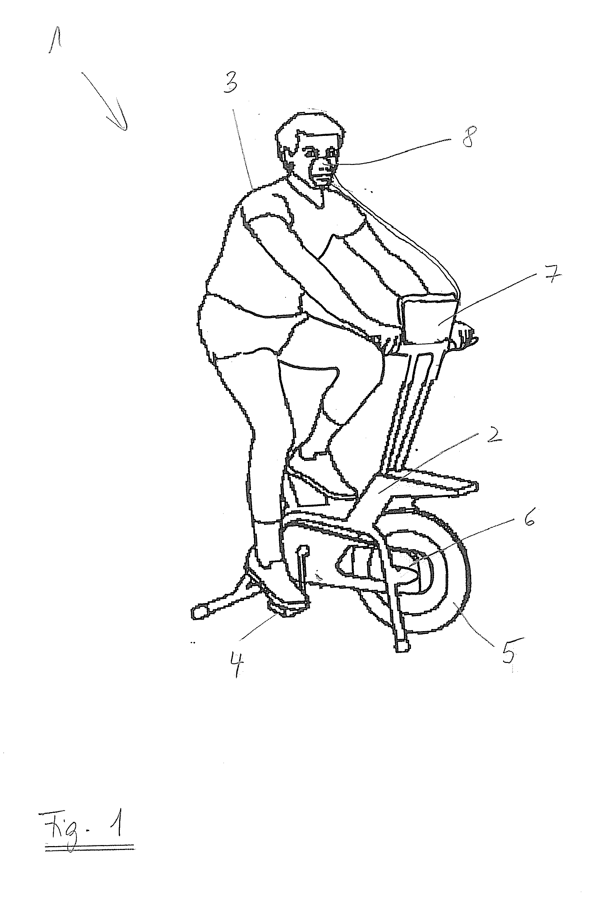

Figur 1 zeigt ein Beispiel für ein Ergometer 1 mit einer in das Ergometer 1 integrierten Vorrichtung 2 zum Einstellen der Belastungsstärke. Bei dem Ergometer 1 handelt es sich um ein Fahrradergometer, bei dem ein Trainierender 3 mit Pedalen 4 eine Schwungscheibe 5 in Rotation versetzt. Die Schwungscheibe 5 bewegt sich gegen die Kraft einer Wirbelstrombremse 6, deren Kraft von der Vorrichtung 2 zum Einstellen der Belastungsstärke geregelt wird.

Die Vorrichtung 2 zum Einstellen der Belastungsstärke ist mit einer Messeinrichtung 7 zur Quantifizierung von Gaskonzentrationen verbunden. Zu der Messeinrichtung gehören nicht gezeigte Messmittel zur Erfassung von Sauerstoff und Kohlenstoffdioxidwerten in einer Atemmaske 8.

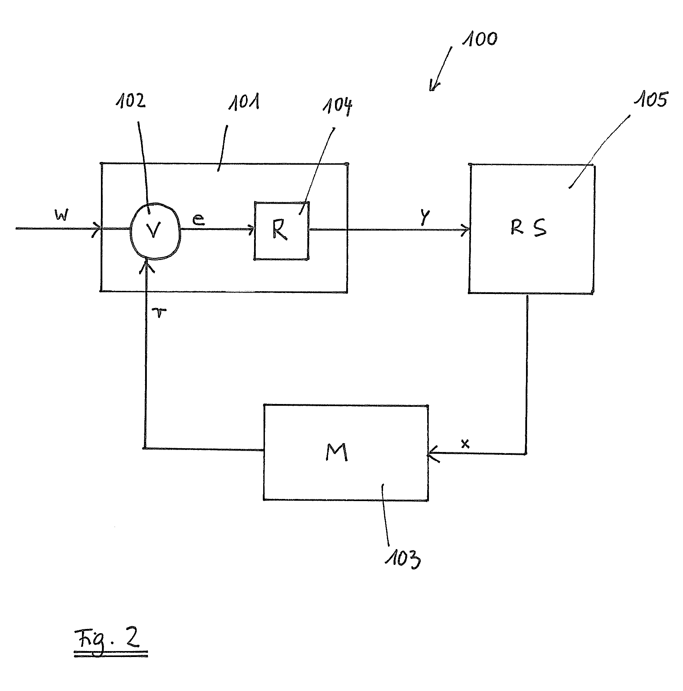

Figur 2 zeigt eine schematische Darstellung eines Regelkreises 100.

Vorgegeben ist eine Führungsgrösse, bzw. ein Sollwert, w. Die Führungsgrösse gibt den Wert an, welcher die Regelgrösse x in vorgegebener Abhängigkeit folgen soll. Es kann sich dabei z.B. um eine vorgegeben Gaskonzentration im Atemgas, um einen bestimmten Sauerstoffverbrauch pro Minute, um ein bestimmtes Verhältnis von Sauerstoff zu Kohlendioxid oder eine andere mit Atemgaskonzentrationen im Zusammenhang stehende Grösse handeln.

Die Regeleinrichtung 101 besteht aus einer Vergleichsstelle 102 und einer Stelleinrichtung 104.

An einer Vergleichsstelle 102 wird eine von einer Messeinrichtung 103 erfasste Rückführgrösse r mit der Führungsgrösse w abgeglichen und eine Regeldifferenz e ermittelt. Die Stelleinrichtung 104 bestimmt aus der Regeldifferenz e die Stellgrösse y. Die Stellgrösse y ist die Ausgangsgrösse der Regeleinrichtung 101 zur Beeinflussung einer Regelstrecke 105. Die Stellgrösse y ist in der Regel ein elektromagnetisches Signal, zum Beispiel ein Strom, der den Widerstand einer Wirbelstrombremse in einem Fahrradergometer festlegt.

Die Regelstrecke 105 ist der Teil des Regelkreises 100, bei dem die Regelgrösse x, von einer Stellgrösse y beeinflusst, auf einem bestimmten Wert gehalten werde soll. Im vorliegenden Fall handelt es ich daher um das Ergometer und den Benutzer.

Auf die Regelstrecke 105 wirkt ausserdem die Störgröße z, deretwegen die Regeleinrichtung 101 überhaupt erst nötig ist. Im vorliegenden Fall setzt sich die Störgrösse z aus dem Einfluss der individuellen Leistung des Benutzers und nicht beeinflussbaren äusseren Parametern, wie der Aussentemperatur, der äusseren Gaskonzentration usw. zusammen.

In Abhängigkeit von der Stellgrösse y, also der Belastungsstärke des Ergometers und von im Wesentlichen der individuellen Leistung des Benutzers herrschen in der Atemluft des Benutzers gewisse Gaskonzentrationen.

Die Messeinrichtung 103, die sowohl Teil der Regelstrecke 105, wie auch der Regeleinrichtung 101 ist, nimmt von der Regelstrecke 105 die Regelgröße x, in diesem Fall die Gaskonzentrationen, als Eingangsgröße auf und verarbeitet sie zur Rückführgröße r weiter, welche sie an die Regeleinrichtung 101 leitet, wodurch der Regelkreis 100 geschlossen ist.

Der CO₂ Gehalt kann beispielsweise mit einem Ultraschall CO₂ Analysator bestimmt werden, wie er von der Firma Ganshorn angeboten wird. Dabei wir das Ultraschallaufzeitverfahren eingesetzt, das die Änderung der Ausbreitungsgeschwindigkeit von Schallwellen relativ zum bewegten Medium zur Bestimmung der der Molmasse verwendet. Ein entsprechendes Verfahren und eine Vorrichtung sind zum Beispiel in EP 1037022 gezeigt.

Der Sauerstoffgehalt kann mit einem Sauerstoffsensor der Firma Dr. Gambert GmbH gemessen werden. Die Sensoren arbeiten nach dem Prinzip der galvanischen Zelle.

Das Training kann beispielsweise an der anaeroben Schwelle, auch Laktatschwelle genannt, durchgeführt werden. Diese Schwelle, bei der die höchstmögliche Belastungsintensität ohne zunehmende Übersäuerung erreicht wird und die Energiebereitstellung rein unter der Verbrennung von Sauerstoff, also "aerob", abläuft, zeichnet sich unter anderem durch das Ende des linearen Zusammenhangs zwischen Sauerstoffverbrauch und ausgeatmetem Kohlendioxid aus.

Für Frauen liegt die Schwelle durchschnittlich bei einem Sauerstoffverbrauch von 11.5 ml/min/kg, für Männer bei 13.0 ml/min/kg. Für ein Training an der anaeroben Schwelle können diese Werte als Sollwerte gewählt werden.

Für ein intensiveres Training können auch kurzzeitig höhere Werte gewählt werden. Gemäss einer Studie von Davos, Doehner, Piepoli, Coats und Anker (Hellenic J Cardiol 44; 243-250,2003) werden im Mittel Werte des Spitzensauerstoffverbrauchs von 18.8 ± 6.4 ml/min/kg erreicht.

Die erreichbaren Werte hängen vom Alter, von der Trainingsstufe und vom allgemeinen gesundheitlichen Zustand ab. Die Festlegung der Sollwerte sollte daher gegebenenfalls unter Rücksprache mit einem Arzt oder einem Fitnesstrainer erfolgen.

## Patentansprüche

1. Vorrichtung zum Einstellen der Belastungsstärke eines Ergometers, **dadurch gekennzeichnet, dass** die Belastungsstärke des Ergometers (1) aufgrund von Gaskonzentrationswerten in der Atemluft regelbar ist.

2. Vorrichtung gemäss Anspruch 1, **dadurch gekennzeichnet, dass** die Vorrichtung (2) eine Messeinrichtung (7; 103) umfasst, welche die Gaskonzentrationswerte in der Atemluft erfasst.

3. Vorrichtung gemäss Anspruch 2, **dadurch gekennzeichnet, dass** die Messeinrichtung (7; 103) mit einer Atemmaske (8) ausgestattet ist

4. Vorrichtung gemäss mindestens einem der vorhergehenden Ansprüche, **dadurch gekennzeichnet, dass** während des Ergometerbetriebs kontinuierlich Gaskonzentrationswerte erfassbar und zur Regelung verwertbar sind.

5. Vorrichtung gemäss mindestens einem der vorhergehenden Ansprüche, **dadurch gekennzeichnet, dass** mindestens Messmittel () zur Bestimmung des Sauerstoffgehalts und/oder des Kohlendioxidgehalts der eingeatmeten und ausgeatmeten Luft vorgesehen sind.

6. Vorrichtung gemäss mindestens einem der vorhergehenden Ansprüche, **dadurch gekennzeichnet, dass** ein Gasumsatz pro Zeitintervall bestimmbar ist.

7. Vorrichtung gemäss mindestens einem der vorhergehenden Ansprüche, **dadurch gekennzeichnet, dass** die Vorrichtung (2) eine Eingabemöglichkeit für Sollwerte und/oder Sollwertkurven und/oder Grenzwerte aufweist.

8. Vorrichtung gemäss mindestens einem der vorhergehenden Ansprüche, **dadurch gekennzeichnet, dass** die Vorrichtung (2) über mindestens eine Anzeige für die Gaskonzentrationswerte und/oder davon abgeleitete Grössen verfügt.

9. Ergometer mit einer Vorrichtung zum Einstellen der Belastungsstärke, insbesondere gemäss mindestens einem der Ansprüche 1-8, **dadurch gekennzeichnet, dass** die Belastungsstärke des Ergometers (1) aufgrund von Gaskonzentrationswerten in der Atemluft regelbar ist.

10. Ergometer gemäss Anspruch 9, **dadurch gekennzeichnet, dass** die Vorrichtung (2) zum Einstellen der Belastungsstärke eine Schnittstelle zu einer Messeinrichtung (7; 103) für Gaskonzentrationswerte in der Atemluft aufweist.

11. Ergometer gemäss mindestens einem der Ansprüche 10 oder 11, **dadurch gekennzeichnet, dass** das Ergometer (1) mit einer Vorrichtung (2) zum Einstellen der Belastungsstärke und einer Messeinrichtung (7; 103) für Gaskonzentrationswerte in der Atemluft ausgestattet ist.

12. Verfahren zur Regelung eines Ergometers **gekennzeichnet durch** folgende Verfahrensschritte (i) Messen von Gaskonzentrationswerten in der Atemluft; (ii) Regeln der Belastungsstärke aufgrund der Gaskonzentrationswerte.

13. Verfahren zur Regelung eines Ergometers gemäss Anspruch 13, **dadurch gekennzeichnet, dass** zusätzlich der Gasumsatz pro Zeitintervall bestimmt wird.
